# EUROPEAN PATENT SPECIFICATION

(11) **EP 1 687 639 B1**
(45) Date of publication and mention of the grant of the patent: **09.05.2007**
(21) Application number: 04821253.4
(22) Date of filing: 21.10.2004
(51) Int. Cl.: G01N 33/68, G01N 33/569

(54) **PROTEOME INTERACTION MAPPING**
PROTEOMWECHSELWIRKUNGSKARTIERUNG
MAPPAGE D'INTERACTION DE PROTEOMES

(30) Priority: 21.10.2003 US 512955 P; 20.10.2004 US 969364
(43) Date of publication of application: 09.08.2006
(73) Proprietor: Athenix Corporation, Research Triangle Park, NC 27709 (US)
(72) Inventor: KOZIEL, Michael, Raleigh, NC 27613 (US); DUCK, Nicholas, B., Apex, NC 27502 (US); KAHN, Theodore, W., Durham, NC 27703 (US); CAROZZI, Nadine, Raleigh, NC 27615 (US); RIEKER, Jennifer, Alexandria, VA 22303 (US)
(74) Representative: Bentham, Andrew
(86) International application number: PCT/US2004/034754
(87) International publication number: WO 2005/073726

(56) References cited:
- MOHAMED S ET AL: "ANTIMYCOTIC SCREENING OF 58 MALAYSIAN PLANTS AGAINST PLANT PATHOGENS" PESTICIDE SCIENCE, ELSEVIER APPLIED SCIENCE PUBLISHER. BARKING, GB, vol. 47, no. 3, 1996, pages 259-264, XP009001842 ISSN: 0031-613X
- DANIEL ANU ET AL: "Denaturation of either Manduca sexta aminopeptidase N or Bacillus thuringiensis Cry1A toxins exposes binding epitopes hidden under nondenaturing conditions" APPLIED AND ENVIRONMENTAL MICROBIOLOGY, vol. 68, no. 5, May 2002 (2002-05), pages 2106-2112, XP002337314 ISSN: 0099-2240
- STEFANELLI S ET AL: "INHIBITORS OF TYPE-I INTERLEUKIN-1 RECEPTOR FROM MICROBIAL METABOLITES" JOURNAL OF ANTIBIOTICS, JAPAN ANTIBIOTICS RESEARCH ASSOCIATION, TOKYO, JP, vol. 50, no. 6, June 1997 (1997-06), pages 484-489, XP008048860 ISSN: 0021-8820
- CORBIN DAVID R ET AL: "The identification and development of proteins for control of insects in genetically modified crops" HORTSCIENCE, vol. 33, no. 4, July 1998 (1998-07), pages 614-617, XP008050103 ISSN: 0018-5345
- WALSH JAMES C: "High throughput, mechanism-based screening techniques for discovering novel agrochemicals" JOURNAL OF BIOMOLECULAR SCREENING, vol. 3, no. 3, October 1998 (1998-10), pages 175-181, XP008050104 ISSN: 1087-0571

## Description

### FIELD OF THE INVENTION

This invention relates to the field of molecular biology. Provided is a method for predicting toxicity of a polypeptide to a pest. This method is useful in identifying polypeptides from organisms such as microbes, fungi, or plants that express proteins with pesticidal activity. It is also useful for determining which proteins are responsible for the activity.

### BACKGROUND OF THE INVENTION

Numerous organisms have been identified which have activity against pests such as insects, fungi, and nematodes. Many microbial strains have been analyzed, and the proteins conferring the pesticidal activity have been isolated. Genes encoding these proteins have been expressed in microbial species and in transgenic plants, which are then resistant to the pest. One commonly used biopesticide is the Gram positive bacterium *Bacillus thuringiensis.* Compositions including *B. thuringiensis* strains or their pesticidal proteins can be used as environmentally acceptable pesticides to control agricultural pests or pest vectors for a variety of human or animal diseases.

The endotoxins of *B. thuringiensis* do not exhibit insecticidal activity until they have been ingested and solubilized in the midgut of the insect. In some cases the ingested protoxin is hydrolyzed by proteases in the insect digestive tract to an active toxic molecule (Höfte and Whiteley (1989) *Microbiol. Rev.* 53:242-255). This toxin binds specifically to apical brush border receptors in the midgut of the target larvae and inserts into the apical membrane creating ion channels or pores, resulting in larval death. The specificity of this interaction has been demonstrated in a Western blot assay (Garczynski *et al.* (1991) *Applied Environ. Micro.* 2816-2820).

Because of the devastation that pests can confer, and the possibility of pests developing resistance to the already identified pesticidal proteins or strains, new compounds with toxic activity towards pests are needed. Current methods of identifying those compounds are time consuming, and some types of pests, such as obligate parasites, are difficult to screen. Therefore, methods for rapidly assaying microbes or other organisms for toxicity to a given pest are desirable.

### SUMMARY OF INVENTION

A method for identifying microbes or other organisms with pesticidal activity is provided. This method is useful to rapidly predict whether or not the organism has toxic activity against a pest. The method comprises binding candidate polypeptides (test polypeptides) expressed by the organism to a substrate, such as a filter or a microtiter plate, contacting the test polypeptides with polypeptides or lipids expressed by a target pest (target substance), and detecting whether the target polypeptides or lipids bind to the test polypeptides. The test proteins may be microbial proteins, such as from *Bacillus thuringiensis,* plant proteins, or proteins from any other organism. The target substance may be obtained from any pest source, including, but not limited to, insects, fungi, nematodes, mites, and ticks. In one embodiment, the target substance is contained in or obtained from brush border membrane vesicles (BBMVs) prepared from the intestinal brush border or the intestinal lining of a target insect or nematode pest. This method can also be used to identify clones or DNA sequences expressing the test polypeptides that are capable of binding to target pest substances, thereby aiding in the identification of novel toxic polypeptides. Additionally, improved toxic substances can be identified from a collection of mutant forms of toxic substances. This method can additionally be used to screen fractionated proteins that bind to target pest substances in order to help identify an active toxic polypeptide or other molecule present in a complex mixture. Novel pesticidal substances can be identified using this method.

The present invention is well suited for high-throughput screening of large numbers of test organisms, each containing large numbers of potential toxins. The invention is also well suited for screening fractions prepared from the test organisms in order to quickly identify a toxin from the complex proteome of the organism. Methods of the present invention are also well-suited for screening expressed libraries of genes from the test organism to quickly identify the gene(s) responsible for toxic activity. In addition, the methods are well-suited for screening collections or libraries of mutants of a particular toxin to identify improved versions of the toxin.

### DESCRIPTION OF FIGURES

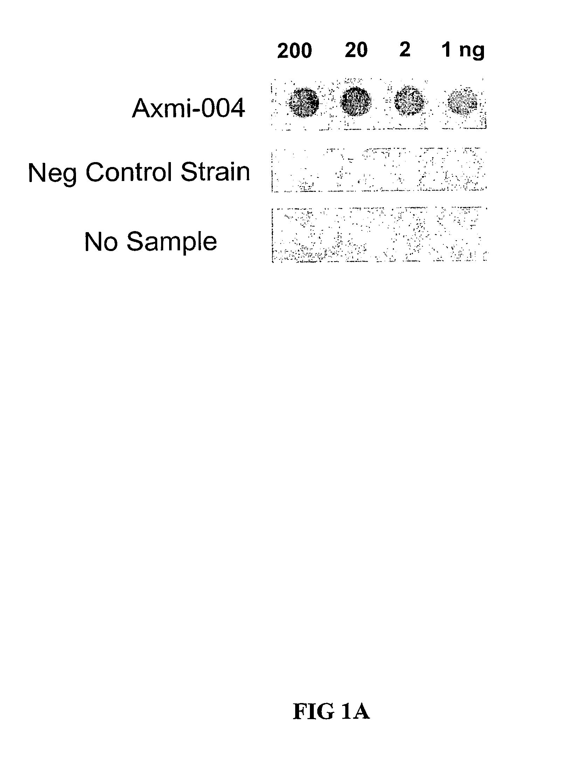

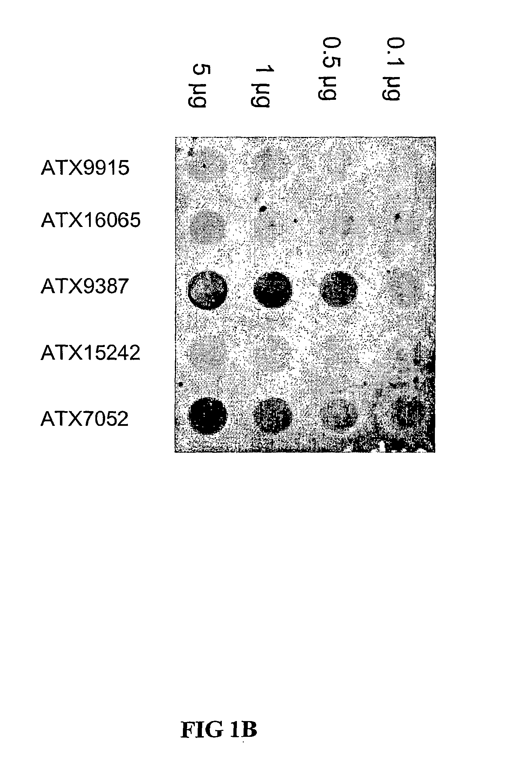

Figure 1A shows the results of a filter-based assay detecting binding of AXMI-004 insecticidal protein to *Ostrinia nubilalis* brush border membrane vesicles. Figure 1B shows the results of a filter-based assay detecting binding of bacterial protein samples to *C*. *elegans* brush border membrane vesicles.

### DETAILED DESCRIPTION

The present invention describes a novel approach for studying toxin-receptor interactions, designed to rapidly identify new toxins that are active against a pest of interest. This invention involves rapidly screening large numbers of polypeptides expressed by microbes or other organisms for their ability to bind target pest polypeptides or lipids (such as proteins or lipids of the intestinal brush border or intestinal lining). This does not require *a priori* knowledge that the screened polypeptides of the microbe or other organism have toxic activity, nor does it require *a priori* knowledge of the nature of the receptor in the target pest. In contrast to working with individually purified toxins, the entire proteome of the microbe or other organism may be screened simultaneously to locate proteins capable of binding to a target substance (such as a polypeptide or lipid associated with the intestinal brush border or intestinal lining). Neither the toxin nor the receptor needs to be identified, separated, or purified in order to determine if an interaction is occurring. In this invention, instead of the target polypeptides or lipids being separated and then immobilized on a filter, the proteomes being screened (test polypeptides) are immobilized (without separation of the individual polypeptides), and the target substances (including, for example, intact brush border membrane vesicles) are allowed to interact with the test polypeptides. Both the test polypeptides and the target substances remain in their native conformation, and protein complexes remain intact, increasing the likelihood that interactions between toxins and receptors will occur as they would *in vivo.* This technique allows numerous organisms to be quickly screened in parallel for polypeptides that interact with target substances from pests including insects, nematodes and fungi. This invention also makes possible the screening of organisms for toxins that will kill pests that are not amenable to *in vitro* bioassays, such as obligate parasites.

Currently employed methods for studying the interaction of toxic polypeptides with target proteins involve immobilizing the target proteins and then allowing the toxic polypeptides to bind (far-Western blots or "ligand blots"). These methods are not well-suited to screening large numbers of samples, and require the use of previously identified toxic polypeptides in a relatively pure form. The present invention improves on these methods in several ways. In the present invention, the entire ensemble of proteins produced by the test organism (the organism producing the test polypeptides) is immobilized, and the entire ensemble of potential target proteins and lipids produced by the target organism (the pest) is allowed to interact with the test polypeptides. This means that no prior knowledge of either the toxin or the target substance is required.

The present invention is drawn to a method for identifying organisms with pesticidal activity. In one embodiment, the method comprises binding the polypeptides expressed by a microbe or other organism (test polypeptides) to a substrate, contacting the substrate with polypeptides or lipids expressed by a pest (target substances), and detecting whether the target substances have bound to the test polypeptides. This assay will allow the rapid identification and isolation of microbial strains or other organisms with effective pesticidal activity. In the same manner, cloned polypeptides and polypeptide libraries from native and transformed bacteria can be readily tested for pesticidal activity.

By "test polypeptide" is intended a polypeptide that is expressed by an organism and that is being tested for pesticidal activity. By "target substance" is intended a polypeptide, lipid, or other molecule that may be capable of binding a test polypeptide.

Numerous pesticidal proteins specifically bind to receptors on BBMVs (Oddou *et al.* (1991) *Eur. J. Biochem.* 202:673-680; Garczynski *et al.* (1991) *Appl. Environ. Microbiol.* 57:2816-2820; MacIntosh *et al.* (1991) *Proc. Natl. Acad. Sci. U.S.A.* 88:8930-8933). Furthermore, the toxicity of delta-endotoxins has been correlated to binding specificity to brush border membrane vesicles (BBMVs) of target insects (Hofmann *et al.* (1988) *Proc. Natl. Acad. Sci, U.S.A.* 85:7844-7848; Van Rie *et al.* (1990) *Appl. Environ. Microbiol.* 56:1378-1385). Typically, to be toxic to an insect, the insecticidal protein, generally an endotoxin, binds to the BBMV of the insect. For purposes of the present invention, the terms "protein" and "polypeptide" are used interchangeably. By "organism" is intended an individual form of life, such as a plant, animal, bacterium, protist or fungus. By "microbe" is intended one or more microscopic organisms, including bacteria, viruses, fungi, and protozoa. Numerous microbes have been identified which have pesticidal activity. These include *Bacillus* (Feitelson (1993) The Bacillus Thuringiensis family tree. *In* Advanced Engineered Pesticides. Marcel Dekker, Inc., New York, N.Y.), *Photorhabdus luminescens* and *Xenorhabdus nematophilus* (ffrench-Constant and Bowen (2000) *Cell Mol. Life Sci.* 57:828-833), *Streptomyces padanus* and other species (Shih *et al.* (2003) *J. Agric. Food Chem.* 51:95-99; Lahdenperä (1991) *Agro-Ind. Hi-Tech.* 2:25-27), *Pseudomonas fluorescens* (Cook (1993) *Annu. Rev. Phytopathol.* 31:53-80), and *Bacillus sphaericus* (Partridge and Berry (2002) *J. Invertebr. Pathol.* 79:135-136).

Other organisms have been identified which express polypeptides that are toxic to pests. Plants have been found that contain compounds with pesticidal activity, including peas (U.S. Patent No. 5,955,082), *Griffonia simplificifolia* (Zhu *et al.* (1996) *Plant Physiol.* 110:195-202), African yam beans (Machuka *et al.* (2000) *Phytochemistry* 53:667-674), mungbean (Chen *et al.* (2002) *J. Agric. Food Chem.* 50:7258-7263), bean (Ishimoto *et al.* (1999) *Biochim. Biophys. Acta* 1432:104-112), and lima bean (Maraes *et al.* (2000) *Braz. J. Med. Biol. Res.* 33:191-198). Fungi, bacteria and plants are known to produce chitinases, which attack chitin, which is a major component of fungal cell walls, and is also found in the gut lining and exoskeleton of insects and nematodes (Samac and Shaw (1991) *The Plant Cell* 3:1063-1072; Collinge *et al.* (1993) *Plant J.* 3:31-40; Herrera-Estrella and Chet (1999) *EXS* 87:171-184; Gooday (1999) *EXS* 87:157-169).

By "pest" is intended insects, fungi, nematodes, mites, ticks, and the like (as described below). Preparations of the proteins expressed by a pest may be obtained by any of numerous methods known in the art, such as by preparing BBMVs from target insect pests (see, for example Wolfersberger *et al.* (1987) *Comp. Biochem. Physiol.* 86A:301-308; Du and Nickerson (1996) *Appl. Env. Micro.* 62:2932-2939). Fungal preps may also be used (see, for example, Ugalde *et al.* (1992) *J. Gen. Microbiol.* 138:2205-2212; Brooks *et al.* (1983) *J. Biol. Chem.* 258:13909-13918; Fuhrmann *et al.* (1974) *Biochim. Biophys. Acta* 363:295-310).

Polypeptides from microbes or other organisms can be isolated by methods known in the art, including, but not limited to, extraction of proteins at different pH values, conventional chromatography and electrophoretic separation. See, for example, Current Protocols in Molecular Biology Vols. 1 and 2, Ausubel *et al.* eds. John Wiley & Sono, NY (1988). If necessary, cells may be disrupted, preferably during the early stages of the protein isolation process, by methods known to those of skill in the art including sonication, grinding, shearing, use of mild detergents, and methods involving a French press. Such methods are often necessary for the isolation of proteins that are membrane bound and/or contained within the cell. See, for example, Scopes (1993) *Protein Purification: Principles and Practice* (3rd ed., Springer-Verlag, NY). In the case of membrane-bound proteins, insoluble components, including, but not limited to, membrane vesicles and other membrane parts may be separated from soluble components of the cell by sedimentation methods such as centrifugation and differential centrifugation. See, for example, Scopes (1993) *Protein Purification: Principles and Practice* (3rd ed., Springer-Verlag, NY).

The substrate used to immobilize microbial proteins may be filter based, such as a nylon or nitrocellulose membrane, or may be a microtiter plate. Plates that can be used include, but are not limited to, plates that are designed to bind large amounts of protein such as Corning High Binding Plates, or plates that are designed to bind proteins by crosslinking, such as Corning Photo-reactive (Universal-BIND^{™}) Binding Plates. The test polypeptides or compounds are bound to the substrate by, for example, electrostatic interactions, hydrophobic interactions, and/or the formation of covalent bonds. The protein samples can be applied to filters by a number of techniques including using a pin tool, using a vacuum manifold, or spotting samples onto the filter with a pipet. While each of these methods is effective, a vacuum manifold may be optimal in that it allows larger volumes of protein sample to be applied to the filter.

Suitable detection markers for detecting bound polypeptides or lipids include endogenous enzymes or compounds within the target preparation, or labels added to the target preparation such as biotin, antigens, radionuclides, enzymes, coenzymes, fluorescers, chemiluminescers, chromogens, enzyme substrates or co-factors, enzyme inhibitors, prosthetic group complexes, free radicals, particles, dyes, including succinimidyl esters of dyes that react with primary amines, and lipophilic dialkylcarbocyanines or other dyes that partition into lipid bilayers, and the like. Markers can be detected by appropriate methods, such as enzyme-linked avidin or streptavidin for biotin, enzyme-linked antibodies for antigens, and appropriate chemical substrates for enzymes. Examples of suitable enzymes that can be used directly as markers, or linked to proteins that bind to markers include horseradish peroxidase, alkaline phosphatase, β-galactosidase, or acetylcholinesterase; examples of suitable fluorescent materials include umbelliferone, fluorescein, fluorescein isothiocyanate, rhodamine, dichlorotriazinylamine fluorescein, dansyl chloride or phycoerythrin; an example of a luminescent material is luminol; examples of bioluminescent materials include luciferase, luciferin, and aequorin; and examples of suitable radioactive material include ¹²⁵I, ¹³¹I, ³⁵S, or ³H. Examples of suitable dyes include succinimidyl esters of dyes such as Alexa Fluor dyes (Molecular Probes, Inc.), or the lipophilic dialkylcarbocyanine fluorescent dyes DiO and DiD (Molecular Probes, Inc.). Many of these compounds can be used to directly label markers, or can be used to label proteins that detect the presence of markers, or can be generated as products of enzyme-catalyzed reactions, where the enzyme is used as a marker or is linked to a protein or compound that detects the presence of a marker.

Enzymes are typically detected by their activity. For example, horseradish peroxidase is usually detected by its ability to convert 3,3 ',5,5 '-tetramethylbenzidine (TMB) to a blue pigment, quantifiable with a spectrophotometer. Other detection systems include biotin and avidin or streptavidin, Ig G and protein A, and the numerous receptor-ligand couples known in the art, in which the receptor can be conjugated to an enzyme.

BBMVs of lepidopteran pests such as European corn borer (ECB) contain the enzyme alkaline phosphatase, which can be visualized using commercially available substrates. If a protein from the BBMV is specifically bound to a test protein, the entire BBMV containing the endogenous alkaline phosphatase binds also, acting as a marker for the interaction. Alkaline phosphatase is not present in the brush border of all pests, so other marker enzymes can also be used to detect the binding of BBMVs to test proteins. It is also possible to add a label to BBMVs from any species. For instance, a biotin marker may be incorporated into the BBMVs, for example by the method described by Altin *et al.* (Altin *et al.* (1995) *Anal. Biochem.* 224:382-389). Incorporation of biotin into the BBMVs can facilitate detection of binding by use of an avidin-conjugated enzyme to recognize biotinylated BBMVs. Methods to detect biotin-containing proteins and lipids are well known in the art.

The proteome of the microbe can be fractionated by, for example, SDS-PAGE, and transferred onto a membrane. The proteins and lipids from the pest organism can be used to bind and identify the protein responsible for the specific interaction. In addition, proteins fractionated in their native conformations, for example by chromatography, can be immobilized on a substrate, and tested for their ability to bind BBMVs or other target preparations, for example by using the methods of the invention. Alternatively, one may detect the fractionated proteins using methods for detecting protein interactions that are well known in the art (see, for example, Sambrook *et al.* (1989) *Molecular Cloning: A Laboratory Manual,* 2nd ed., Cold Spring Harbor Laboratory Press, Plainview, New York). The gene that codes for a specific protein can then be identified by cloning the gene for this protein based on its amino acid sequence and transforming a bacterial host such as *E*. *coli.* Cloning methods are well known in the art (see Sambrook *et al., supra*)*.* After expressing the gene, the resulting protein can be tested in this assay to confirm its ability to bind to the target pest proteome. Alternatively, a library can be prepared from the genome of the microbe, and can be expressed, for instance in *E*. *coli,* and screened for proteins that bind the target substances. Similarly, once a test protein has been identified, a collection or library of mutants of the protein can be tested to identify mutants with improved binding characteristics.

Once a specific interaction is obtained, the microbial strain or individual proteins from the strain can be used in a bioassay against the target pest. Numerous bioassay methods are available, including, but not limited to, Marrone *et al.* (1985) *J. of Economic Entomology* 78:290-293 and Czapla and Lang (1990) *J. Econ. Entomol. 83*:2480-2485.

### Pesticidal Genes and Transgenic Organisms

The genes encoding the proteins responsible for conferring pesticidal activity in the microbes can be identified, cloned and expressed in transgenic organisms by methods known by one of skill in the art.

Any combination of methods may be utilized to purify proteins having pesticidal properties. As the protocol is being formulated, pesticidal activity is determined after each purification step to assure the presence of the toxin of interest. Methods are available in the art to assay for pesticidal activity. Generally the protein is mixed and used in feeding assays. See, for example, Marrone *et al.* (1985) *J. of Economic Entomology 78*:290-293 and Czapla and Lang (1990) *J. Econ. Entomol. 83*:2480-2485. Such purification steps will result in a substantially purified protein fraction.

Once the purified protein is isolated, the protein, or the polypeptides of which it is comprised, can be characterized and sequenced by standard methods known in the art. For example, the purified protein, or the polypeptides of which it is comprised, may be fragmented as with cyanogen bromide, or with proteases such as papain, chymotrypsin, trypsin, lysyl-C endopeptidase, etc. (Oike *et al.* (1982) *J. Biol Chem.* 257:9751-9758; Liu *et al.* (1983) *Int. J. Pept. Protein Res. 21*:209-215). The resulting peptides are separated, preferably by HPLC, or by resolution of gels and electroblotting onto PVDF membranes, and subjected to amino acid sequencing. To accomplish this task, the peptides are preferably analyzed by automated sequencers. It is recognized that N-terminal, C-terminal, or internal amino acid sequences can be determined. From the amino acid sequence of the purified protein, a nucleotide sequence can be synthesized which can be used as a probe to aid in isolation of the gene encoding the pesticidal protein.

Antibodies can be prepared against substantially pure preparations of the protein; (see, for example, Radka *et al.* (1983) *J. Immunol.* 128:2804; Radka *et al.* (1984) *Immunogenetics* 19:63.) Antibodies made against substantially pure preparations of the pesticidal protein may be used to identify and isolate pesticidal proteins and protein coding sequences in other bacteria and in other organisms.

It is recognized that there are alternative methods available to obtain the nucleotide and amino acid sequences of the present proteins. For example, to obtain the nucleotide sequence encoding a pesticidal protein, a genomic library may be prepared from isolated genomic DNA of a strain determined to have pesticidal activity by the method of the invention. Such a library may be screened for clones expressing the pesticidal protein. Expression of the pesticidal protein may be determined by, for example, monitoring pesticidal activity or by using immunological methods involving the detection of the pesticidal protein with cross-reactive antibodies. From larger clones, smaller subclones can be made and tested for activity. In this manner, clones which express an active pesticidal protein can be identified and used to determine the nucleotide sequence of the gene using standard procedures. Then, an amino acid sequence can be deduced for the protein. For general molecular methods, see, for example, Sambrook *et al.* (1989) *Molecular Cloning: A Laboratory Manual* (2nd ed., Cold Spring Harbor Laboratory Press, Plainview, New York), and the references cited therein. Once the nucleotide sequences encoding the pesticidal proteins have been isolated, they can be manipulated and used to transform genetically a variety of organisms, including, but not limited to, plants and microorganisms. The transformed plants and microorganisms produce pesticidal proteins and find use in methods for impacting pests and as sources of pesticidal proteins for use in pesticidal compositions.

A number of methods are available for introducing a gene expressing the pesticidal protein into the microorganism host under conditions which allow for stable maintenance and expression of the gene. For example, expression cassettes can be constructed which include the pesticidal protein operably linked with the transcriptional and translational regulatory signals for expression of the pesticidal protein, and a DNA sequence homologous with a sequence in the host organism, whereby integration will occur, and/or a replication system which is functional in the host, whereby integration or stable maintenance will occur. The expression cassette may additionally contain selectable marker genes.

Transformation protocols as well as protocols for introducing nucleotide sequences into plants may vary depending on the type of plant or plant cell, i.e., monocot or dicot, targeted for transformation. Suitable methods of introducing nucleotide sequences into plant cells and subsequent insertion into the plant genome include microinjection (Crossway *et al.* (1986) *Biotechniques 4*:320-334), electroporation (Riggs *et al.* (1986) *Proc. Natl. Acad. Sci. USA 83*:5602-5606, *Agrobacterium*-mediated transformation (Townsend *et al.,* U.S. Pat No. 5,563,055), direct gene transfer (Paszkowski *et al.* (1984) *EMBO J. 3*:2717-2722), and ballistic particle acceleration (see, for example, Sanford *et al.,* U.S. Patent No. 4,945,050; Tomes *et al.* (1995) "Direct DNA Transfer into Intact Plant Cells via Microprojectile Bombardment," in *Plant Cell, Tissue, and Organ Culture: Fundamental Methods,* ed. Gamborg and Phillips (Springer-Verlag, Berlin); and McCabe *et al.* (1988) *Biotechnology 6*:923-926). Also see Weissinger *et al.* (1988) *Ann. Rev. Genet. 22*:421-477; Sanford *et al.* (1987) *Particulate Science and Technology 5*:27-37 (onion); Christou *et al.* (1988) *Plant Physiol. 87*:671-674 (soybean); McCabe *et al.* (1988) *Bio*/*Technology 6*:923-926 (soybean); Finer and McMullen (1991) *In Vitro Cell Dev. Biol.* 27P:175-182 (soybean); Singh *et al.* (1998) *Theor. Appl. Genet. 96*:319-324 (soybean); Datta *et al.* (1990) *Biotechnology 8*:736-740 (rice); Klein *et al.* (1988) *Proc. Natl. Acad. Sci. USA 85*:4305-4309 (maize); Klein *et al.* (1988) *Biotechnology 6*:559-563 (maize); Tomes, U.S. Patent No. 5,240,855; Buising *et al.,* U.S. Patent Nos. 5,322,783 and 5,324,646; Tomes *et al. (1995)* "Direct DNA Transfer into Intact Plant Cells via Microprojectile Bombardment," in *Plant Cell, Tissue, and Organ Culture: Fundamental Methods,* ed. Gamborg (Springer-Verlag, Berlin) (maize); Klein *et al.* (1988) *Plant Physiol. 91*:440-444 (maize); Fromm *et al.* (1990) *Biotechnology 8*:833-839 (maize); Hooykaas-Van Slogteren *et al.* (1984) *Nature (London) 311*:763-764; Bowen *et al.,* U.S. Patent No. 5,736,369 (cereals); Bytebier *et al.* (1987) *Proc. Natl. Acad. Sci. USA 84*:5345-5349 (Liliaceae); De Wet *et al.* (1985) in *The Experimental Manipulation of Ovule Tissues,* ed. Chapman *et al.* (Longman, New York), pp. 197-209 (pollen); Kaeppler *et al.* (1990) *Plant Cell Reports 9*:415-418 and Kaeppler *et al.* (1992) *Theor. Appl. Genet. 84*:560-566 (whisker-mediated transformation); D'Halluin *et al.* (1992) *Plant Cell 4*:1495-1505 (electroporation); Li *et al.* (1993) *Plant Cell Reports 12*:250-255 and Christou and Ford (1995) *Annals of Botany 75*:407-413 (rice); Osjoda *et al.* (1996) *Nature Biotechnology 14*:745-750 (maize via *Agrobacterium tumefaciens*).

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent. The shoots are then transferred to a selective rooting medium for recovering rooted shoot or plantlet. The transgenic plantlet then grows into a mature plant and produces fertile seeds (e.g. Hiei *et al.* (1994) *The Plant Journal 6*: 271-282; Ishida *et al.* (1996) *Nature Biotechnology* 14: 745-750). Explants are typically transferred to a fresh supply of the same medium and cultured routinely. A general description of the techniques and methods for generating transgenic plantlets are found in Ayres and Park (1994) *Critical Reviews in Plant Science* 13: 219-239 and Bommineni and Jauhar (1997) *Maydica* 42: 107-120. Since the transformed material contains many cells; both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants.

The cells that have been transformed may be grown into plants in accordance with conventional ways. See, for example, McCormick *et al.* (1986) *Plant Cell Reports 5*:81-84. These plants may then be grown, and either pollinated with the same transformed strain or different strains, and the resulting hybrid having constitutive expression of the desired phenotypic characteristic identified. Two or more generations may be grown to ensure that constitutive expression of the desired phenotypic characteristic is stably maintained and inherited and then seeds harvested to ensure constitutive expression of the desired phenotypic characteristic has been achieved.

Pesticidal proteins discovered by the methods of the invention may be used for protecting agricultural crops and products from pests. Alternatively, a gene encoding a pesticidal protein may be introduced via a suitable vector into a microorganism. A variety of microorganisms may be used including, but not limited to, bacteria, yeast and other microbial fungi, nematodes and viruses. The transformed microorganism may be applied to the environment of insect pests. Microorganisms may be selected which are known to occupy the "phytosphere" (phylloplane, phyllosphere, rhizosphere, and/or rhizoplane) of one or more crops of interest. These microorganisms are selected so as to be capable of successfully competing in the particular environment with the wild-type microorganisms, provide for stable maintenance and expression of the gene expressing the polypeptide insecticide and, desirably, provide for improved protection of the pesticide from environmental degradation and inactivation. In another embodiment of the invention a pesticidal gene may be introduced into a microorganism for the purpose of producing the pesticidal protein encoded by the pesticidal gene. The transformed microorganism may be used directly or as a component of pesticidal compositions in methods of impacting insect pests. Alternatively, the transformed microorganism may be used as a source for the isolation of the pesticidal protein.

### Evaluation of Plant Transformation

Following introduction of heterologous foreign DNA into plant cells, the transformation or integration of heterologous gene in the plant genome is confirmed by various methods such as analysis of nucleic acids, proteins and metabolites associated with the integrated gene.
PCR Analysis: PCR analysis is a rapid method to screen transformed cells, tissue or shoots for the presence of incorporated gene at the earlier stage before transplanting into the soil (Sambrook and Russell, 2001. *Molecular Cloning: A Laboratory Manual.* Cold Spring Harbor Laboratory Press, Cold Spring Harbor, NY). PCR is carried out using oligonucleotide primers specific to the gene of interest or *Agrobacterium* vector background, etc.
Southern Analysis: Plant transformation is confirmed by Southern blot analysis of genomic DNA (Sambrook and Russell, 2001, supra). In general, total DNA is extracted from the transformant, digested with appropriate restriction enzymes, fractionated in an agarose gel and transferred to a nitrocellulose or nylon membrane. The membrane or "blot" then is probed with, for example, radiolabeled ³²P target DNA fragment to confirm the integration of introduced gene in the plant genome according to standard techniques (Sambrook and Russell, 2001, supra).
Northern Analysis: RNA is isolated from specific tissues of transformant, fractionated in a formaldehyde agarose gel, blotted onto a nylon filter according to standard procedures that are routinely used in the art (Sambrook and Russell, 2001, supra). Expression of RNA encoded by the pesticidal gene is then tested by hybridizing the filter to a radioactive probe derived from the pesticidal gene, by methods known in the art (Sambrook and Russell, 2001, supra).
Western blot and Biochemical assays: Western blot and biochemical assays and the like may be carried out on the transgenic plants to confirm the presence of protein encoded by the pesticidal gene by standard procedures (Sambrook and Russell, 2001, supra) using antibodies that bind to one or more epitopes present on the pesticidal protein.

### Pesticidal Activity in Plants

In another aspect of the invention, one may generate transgenic plants expressing a pesticidal protein that has pesticidal activity. Methods described above by way of example may be utilized to generate transgenic plants, but the manner in which the transgenic plant cells are generated is not critical to this invention. Methods known or described in the art such as *Agrobacterium-*mediated transformation, biolistic transformation, and non-particle-mediated methods may be used at the discretion of the experimenter. Plants expressing the pesticidal protein may be isolated by common methods described in the art, for example by transformation of callus, selection of transformed callus, and regeneration of fertile plants from such transgenic callus. In such process, one may use any gene as a selectable marker so long as its expression in plant cells confers ability to identify or select for transformed cells.

A number of markers have been developed for use with plant cells, such as resistance to chloramphenicol, the aminoglycoside G418, hygromycin, or the like. Other genes that encode a product involved in chloroplast metabolism may also be used as selectable markers. For example, genes that provide resistance to plant herbicides such as glyphosate, bromoxynil, or imidazolinone may find particular use. Such genes have been reported (Stalker *et al.* (1985) *J. Biol. Chem.* 263:6310-6314 (bromoxynil resistance nitrilase gene); and Sathasivan *et al.* (1990) *Nucl. Acids Res.* 18:2188 (AHAS imidazolinone resistance gene).

Fertile plants expressing the gene of interest may be tested for pesticidal activity, and the plants showing optimal activity selected for further breeding. Methods are available in the art to assay for pest activity. Generally, the protein is mixed and used in feeding assays. See, for example Marrone *et al.* (1985) *J. of Economic Entomology* 78:290-293.

### Use in Pesticidal Control

General methods for employing the pesticidal proteins identified by the methods of the invention in pesticide control or in engineering other organisms as pesticidal agents are known in the art. See, for example U.S. Patent No. 5,039,523 and EP 0480762A2.

The microorganisms which have been genetically altered to contain the pesticidal gene and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticide is produced by introducing a pesticidal gene into a cellular host. Expression of the pesticidal gene results, directly or indirectly, in the intracellular production and maintenance of the pesticide. In one aspect of this invention, these cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein. Alternatively, one may formulate the cells expressing the pesticidal genes such as to allow application of the resulting material as a pesticide.

The active ingredients of the present invention are normally applied in the form of compositions and can be applied to the crop area or plant to be treated, simultaneously or in succession, with other compounds. These compounds can be fertilizers, weed killers, cryoprotectants, surfactants, detergents, pesticidal soaps, dormant oils, polymers, and/or time-release or biodegradable carrier formulations that permit long-term dosing of a target area following a single application of the formulation. They can also be selective herbicides, chemical insecticides, virucides, microbicides, amoebicides, pesticides, fungicides, bacteriocides, nematocides, mollusocides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. Likewise the formulations may be prepared into edible "baits" or fashioned into pest "traps" to permit feeding or ingestion by a target pest of the pesticidal formulation.

Methods of applying a pesticidal protein identified by the methods of the present invention or an agrochemical composition containing a pesticidal protein identified by the methods of the present invention are leaf application, seed coating and soil application. The number of applications and the rate of application depend on the intensity of infestation by the corresponding pest.

The composition may be formulated as a powder, dust, pellet, granule, spray, emulsion, colloid, solution, or such like, and may be preparable by such conventional means as desiccation, lyophilization, homogenation, extraction, filtration, centrifugation, sedimentation, or concentration of a culture of cells comprising the polypeptide. In all such compositions that contain at least one such pesticidal polypeptide, the polypeptide may be present in a concentration of from about 1% to about 99% by weight.

Lepidopteran or coleopteran pests may be killed or reduced in numbers in a given area by using a pesticidal protein identified by the methods of the invention, or a composition comprising a pesticidal protein identified by the methods of the invention may be prophylactically applied to an environmental area to prevent infestation by a susceptible pest. Preferably the pest ingests, or is contacted with, a pesticidally-effective amount of the polypeptide. By "pesticidally-effective amount" is intended an amount of the pesticide that is able to bring about death to at least one pest, or to noticeably reduce pest growth, feeding, or normal physiological development. This amount will vary depending on such factors as, for example, the specific target pests to be controlled, the specific environment, location, plant, crop, or agricultural site to be treated, the environmental conditions, and the method, rate, concentration, stability, and quantity of application of the pesticidally-effective polypeptide composition. The formulations may also vary with respect to climatic conditions, environmental considerations, and/or frequency of application and/or severity of pest infestation.

The pesticide compositions described may be made by formulating the bacterial cell, crystal and/or spore suspension, or isolated protein component with the desired agriculturally-acceptable carrier. The compositions may be formulated prior to administration in an appropriate means such as lyophilized, freeze-dried, desiccated, or in an aqueous carrier, medium or suitable diluent, such as saline or other buffer. The formulated compositions may be in the form of a dust or granular material, or a suspension in oil (vegetable or mineral), or water or oil/water emulsions, or as a wettable powder, or in combination with any other carrier material suitable for agricultural application. Suitable agricultural carriers can be solid or liquid and are well known in the art. The term "agriculturally-acceptable carrier" covers all adjuvants, inert components, dispersants, surfactants, tackifiers, binders, etc. that are ordinarily used in pesticide formulation technology; these are well known to those skilled in pesticide formulation. The formulations may be mixed with one or more solid or liquid adjuvants and prepared by various means, e.g., by homogeneously mixing, blending and/or grinding the pesticidal composition with suitable adjuvants using conventional formulation techniques. Suitable formulations and application methods are described in U.S. Patent No. 6,468,523.

### Target Pests

Target pests for use in the method of the invention include but are not limited to, insects, fungi, nematodes, mites, ticks, and the like. "Pesticidal activity" is the property of a substance or organism that can be measured by, but is not limited to, mortality, weight loss, attraction, repellency and other behavioral and physical changes after feeding and exposure for an appropriate length of time. "Pesticidal proteins" are proteins which display pesticidal activity by themselves or in combination with other proteins.

Specific fungal pathogens for the major crops include: Soybeans: *Phytophthora megasperma* fsp. *glycinea, Macrophomina phaseolina, Rhizoctonia solani, Sclerotinia sclerotiorum, Fusarium oxysporum, Diaporthe phaseolorum* var. *sojae (Phomopsis sojae), Diaporthe phaseolorum* var. *caulivora, Sclerotium rolfsii, Cercospora kikuchii, Cercospora sojina, Peronospora manshurica, Colletotrichum dematium (Colletotichum truncatum), Corynespora cassiicola, Septoria glycines, Phyllosticta sojicola, Alternaria alternata, Pseudomonas syringae* p.v. *glycinea, Xanthomonas campestris* p.v. *phaseoli, Microsphaera diffusa, Fusarium semitectum, Phialophora gregata, Glomerella glycines, Phakopsora pachyrhizi, Pythium aphanidermatum, Pythium ultimum, Pythium debaryanum, Heterodera glycines, Fusarium solani;* Canola: *Albugo candida, Alternaria brassicae, Leptosphaeria maculans, Rhizoctonia solani, Sclerotinia sclerotiorum, Mycosphaerella brassiccola, Pythium ultimum, Peronospora parasitica, Fusarium roseum, Alternaria alternata*; Alfalfa: *Clavibater michiganese subsp. insidiosum, Pythium ultimum, Pythium irregulare, Pythium splendens, Pythium debaryanum, Pythium aphanidermatum, Phytophthora megasperma, Peronospora trifoliorum, Phoma medicaginis* var. *medicaginis, Cercospora medicaginis, Pseudopeziza medicaginis, Leptotrochila medicaginis, Fusar-atrum, Xanthomonas campestris* p.v. *alfalfae, Aphanomyces euteiches, Stemphylium herbarum, Stemphylium alfalfae;* Wheat: *Pseudomonas syringae* p.v. *atrofaciens, Urocystis agropyri, Xanthomonas campestris* p.v. *translucens, Pseudomonas syringae* p.v. *syringae, Alternaria alternata, Cladosporium herbarum, Fusarium graminearum, Fusarium avenaceum, Fusarium culmorum, Ustilago tritici, Ascochyta tritici, Cephalosporium gramineum, Collotetrichum graminicola, Erysiphe graminis* f.sp. *tritici, Puccinia graminis* f.sp. *tritici, Puccinia recondita* f.sp. *tritici, Puccinia striiformis, Pyrenophora tritici-repentis, Septoria nodorum, Septoria tritici, Septoria avenae, Pseudocercosporella herpotrichoides, Rhizoctonia solani, Rhizoctonia cerealis, Gaeumannomyces graminis* var. *tritici, Pythium aphanidermatum, Pythium arrhenomanes, Pythium ultimum, Bipolaris sorokiniana, Claviceps purpurea, Tilletia tritici, Tilletia laevis, Ustilago tritici, Tilletia indica, Rhizoctonia solani, Pythium arrhenomannes, Pythium gramicola, Pythium aphanidermatum*; Sunflower: *Plasmophora halstedii, Sclerotinia sclerotiorum,* Aster Yellows, *Septoria helianthi, Phomopsis helianthi, Alternaria helianthi, Alternaria zinniae, Botrytis cinerea, Phoma macdonaldii, Macrophomina phaseolina, Erysiphe cichoracearum, Rhizopus oryzae, Rhizopus arrhizus, Rhizopus stolonifer, Puccinia helianthi, Verticillium dahliae, Erwinia carotovorum* pv. *carotovora, Cephalosporium acremonium, Phytophthora cryptogea, Albugo tragopogonis; orobanche cumana* Corn: *Fusarium moniliforme* var. *subglutinans, Erwinia stewartii, Fusarium moniliforme, Gibberella zeae (Fusarium graminearum), Stenocarpella maydi (Diplodia maydis), Pythium irregulare, Pythium debaryanum, Pythium graminicola, Pythium splendens, Pythium ultimum, Pythium aphanidermatum, Aspergillus flavus, Bipolaris maydis* O, T *(Cochliobolus heterostrophus), Helminthosporium carbonum* I, II & III *(Cochliobolus carbonum), Exserohilum turcicum* I, II & III, *Helminthosporium pedicellatum, Physoderma maydis, Phyllosticta maydis, Kabatie-maydis, Cercospora sorghi, Ustilago maydis, Puccinia sorghi, Puccinia polysora, Macrophomina phaseolina, Penicillium oxalicum, Nigrospora oryzae, Cladosporium herbarum, Curvularia lunata, Curvularia inaequalis, Curvularia pallescens, Clavibacter michiganense* subsp. *nebraskense, Trichoderma viride, Claviceps sorghi, Pseudonomas avenae, Erwinia chrysanthemi* pv. *zea, Erwinia corotovora, Cornstunt spiroplasma, Diplodia macrospora, Sclerophthora macrospora, Peronosclerospora sorghi, Peronosclerospora philippinensis, Peronosclerospora maydis, Peronosclerospora sacchari, Spacelotheca reiliana, Physopella zeae, Cephalosporium maydis, Caphalosporium acremonium*; Sorghum: *Exserohilum turcicum, Colletotrichum graminicola (Glomerella graminicola), Cercospora sorghi, Gloeocercospora sorghi, Ascochyta sorghina, Pseudomonas syringae* p.v. *syringae, Xanthomonas campestris* p.v. *holcicola, Pseudomonas andropogonis, Puccinia purpurea, Macrophomina phaseolina, Perconia circinata, Fusarium moniliforme, Alternaria alternate, Bipolaris sorghicola, Helminthosporium sorghicola, Curvularia lunata, Phoma insidiosa, Pseudomonas avenae (Pseudomonas alboprecipitans), Ramulispora sorghi, Ramulispora sorghicola, Phyllachara sacchari, Sporisorium reilianum (Sphacelotheca reiliana), Sphacelotheca cruenta, Sporisorium sorghi, Claviceps sorghi, Rhizoctonia solani, Acremonium strictum, Sclerophthona macrospora, Peronosclerospora sorghi, Peronosclerospora philippinensis, Sclerospora graminicola, Fusarium graminearum, Fusarium oxysporum, Pythium arrhenomanes, Pythium graminicola,* etc.

Insect pests include insects selected from the orders *Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera,* etc., particularly *Coleoptera, Lepidoptera,* and *Diptera.* Insect pests of the invention for the major crops include: Maize: *Ostrinia nubilalis,* European com borer; *Agrotis ipsilon,* black cutworm; *Helicoverpa zea,* com earworm; *Spodoptera frugiperda,* fall armyworm; *Diatraea grandiosella,* southwestern corn borer; *Elasmopalpus lignosellus,* lesser cornstalk borer; *Diatraea saccharalis,* surgarcane borer; *Diabrotica virgifera,* western corn rootworm; *Diabrotica longicornis barberi,* northern corn rootworm; *Diabrotica undecimpunctata howardi,* southern corn rootworm; *Melanotus* spp., wireworms; *Cyclocephala borealis,* northern masked chafer (white grub); *Cyclocephala immaculata,* southern masked chafer (white grub); *Popillia japonica,* Japanese beetle; *Chaetocnema pulicaria,* corn flea beetle; *Sphenophorus maidis,* maize billbug; *Rhopalosiphum maidis,* corn leaf aphid; *Anuraphis maidiradicis,* corn root aphid; *Blissus leucopterus leucopterus,* chinch bug; *Melanoplus femurrubrum,* redlegged grasshopper; *Melanoplus sanguinipes,* migratory grasshopper; *Hylemya platura,* seedcorn maggot; *Agromyza parvicornis,* corn blot leafminer; *Anaphothrips obscrurus,* grass thrips; *Solenopsis milesta,* thief ant; *Tetranychus urticae,* twospotted spider mite; Sorghum: *Chilo partellus,* sorghum borer; *Spodoptena frugiperda,* fall armyworm; *Helicoverpa zea,* corn earworm; *Elasmopalpus lignosellus,* lesser cornstalk borer; *Feltia subterranea,* granulate cutworm; *Phylophaga crinita,* white grub; *Eleodes, Conoderus,* and *Aeolus* spp., wireworms; *Oulema melanopus,* cereal leaf beetle; *Chaetocnema pulicaria,* corn flea beetle; *Sphenophorus maidis,* maize billbug; *Rhopalosiphum maidis;* corn leaf aphid; *Sipha flava,* yellow sugarcane aphid; *Blissus leucopterus leucopterus,* chinch bug; *Contarinia sorghicola,* sorghum midge; *Tetranychus cinnabarinus,* carmine spider mite; *Tetranychus urticae,* twospotted spider mite; Wheat: *Pseudaletia unipunctata,* army worm; *Spodoptera frugiperda,* fall armyworm; *Elasmopalpus lignosellus,* lesser cornstalk borer; *Agrotis orthogonia,* western cutworm; *Elasmopalpus lignosellus,* lesser cornstalk borer; *Oulema melanopus,* cereal leaf beetle; *Hypera punctata,* clover leaf weevil; *Diabrotica undecimpunctata howardi,* southern corn rootworm; Russian wheat aphid; *Schizaphis graminum,* greenbug; *Macrosiphum avenae,* English grain aphid; *Melanoplus femurrubrum,* redlegged grasshopper; *Melanoplus differentialis,* differential grasshopper; *Melanoplus sanguinipes,* migratory grasshopper; *Mayetiola destructor,* Hessian fly; *Sitodiplosis mosellana,* wheat midge; *Meromyza americana,* wheat stem maggot; *Hylemya coarctata,* wheat bulb fly; *Frankliniella fusca,* tobacco thrips; *Cephus cinctus,* wheat stem sawfly; *Aceria tulipae,* wheat curl mite; Sunflower: *Suleima helianthana,* sunflower bud moth; *Homoeosoma electellum,* sunflower moth; *zygogramma exclamationis,* sunflower beetle; *Bothyrus gibbosus,* carrot beetle; *Neolasioptera murtfeldtiana,* sunflower seed midge; Cotton: *Heliothis virescens,* cotton budworm; *Helicoverpa zea,* cotton bollworm; *Spodaptera exigua,* beet armyworm; *Pectinophora gossypiella,* pink bollworm; *Anthonomus grandis,* boll weevil; *Aphis gossypii,* cotton aphid; *Pseudatomoscelis seriatus,* cotton fleahopper; *Trialeurodes abutilonea,* bandedwinged whitefly; *Lygus lineolaris,* tarnished plant bug; *Melanoplus femurrubrum,* redlegged grasshopper; *Melanoplus differentialis,* differential grasshopper; *Thrips tabaci,* onion thrips; *Franklinkiella fusca,* tobacco thrips; *Tetranychus cinnabarinus,* carmine spider mite; *Tetranychus urticae,* twospotted spider mite; Rice: *Diatraea saccharalis,* sugarcane borer; *Spodoptera frugiperda,* fall armyworm; *Helicoverpa zea,* corn earworm; *Colaspis brunnea,* grape colaspis; *Lissorhoptrus oryzophilus,* rice water weevil; *Sitophilus oryzae,* rice weevil; *Nephotettix nigropictus,* rice leafhopper; *Blissus leucopterus leucopterus,* chinch bug; *Acrosternum hilare,* green stink bug; Soybean: *Pseudoplusia includens,* soybean looper; *Anticarsia gemmatalis,* velvetbean caterpillar; *Plathypena scabra,* green cloverworm; *Ostrinia nubilalis,* European corn borer; *Agrotis ipsilon,* black cutworm; *Spodoptera exigua,* beet armyworm; *Heliothis virescens,* cotton budworm; *Helicoverpa zea,* cotton bollworm; *Epilachna varivestis,* Mexican bean beetle; *Myzus persicae,* green peach aphid; *Empoasca fabae,* potato leafhopper; *Acrosternum hilare,* green stink bug; *Melanoplus femurrubrum,* redlegged grasshopper; *Melanoplus differentialis,* differential grasshopper; *Hylemya platura,* seedcorn maggot; *Sericothrips variabilis,* soybean thrips; *Thrips tabaci,* onion thrips; *Tetranychus turkestani,* strawberry spider mite; *Tetranychus urticae,* twospotted spider mite; Barley: *Ostrinia nubilalis,* European corn borer; *Agrotis ipsilon,* black cutworm; *Schizaphis graminum,* greenbug; *Blissus leucopterus leucopterus,* chinch bug; *Acrosternum hilare,* green stink bug; *Euschistus servus,* brown stink bug; *Delia platura,* seedcorn maggot; *Mayetiola destructor,* Hessian fly; *Petrobia latens,* brown wheat mite; Oil Seed Rape: *Brevicoryne brassicae,* cabbage aphid; *Phyllotreta cruciferae,* Flea beetle; *Mamestra configurata,* Bertha armyworm; *Plutella xylostella,* Diamond-back moth; *Delia* ssp., Root maggots.

Nematodes include root-knot, cyst, and lesion nematodes, including *Heterodera* spp., *Meloidogyne* spp., and *Globodera* spp.; particularly members of the cyst nematodes, including, but not limited to, *Heterodera glycines* (soybean cyst nematode); *Heterodera schachtii* (beet cyst nematode); *Heterodera avenae* (cereal cyst nematode); and *Globodera rostochiensis* and *Globodera pailida* (potato cyst nematodes). Lesion nematodes include *Pratylenchus* spp.

Mammalian parasites include protozoa, such as the flagellates, the cilliates, the amoeba, and the sporozoa; ectoparasites such as fleas, lice, mites, and ticks; helminthes such as nematodes, cestodes, and trematodes. Nematodes include filariid, ascarid, strongyle, and trichostrongyle nematodes, such as those in the genera *Acanthocheilonema, Aelurostrongylus, Ancylostoma, Angiostrongylus, Ascaris, Brugia, Bunostomum, Dictyocaulus, Dioctophyme, Dipetalonema, Dirofilaria, Dracunculus, Filaroides, Lagochilascaris, Loa, Mansonella, Muellerius, Necator, Onchocerca, Parafilaria, Parascaris, Protostrongylus, Setaria, Stephanofilaria, Strongyloides, Strongylus, Thelazia, Toxascaris, Toxocara, Trichinella, Uncinaria,* and *Wisclaereria.*

Throughout the specification the word "comprising," or variations such as "comprises" or "comprised," will be understood to imply the inclusion of a stated element, interger or step, or group of elements, integers or steps, but not the exclusion of any other element, integer or step, or group of elements, integers or steps.

The following examples are offered by way of illustration and not by way of limitation.

### EXPERIMENTAL

### Example 1. Preparation of Test Samples

To prepare culture supernatant samples and high pH-solubilized samples, bacterial cultures were centrifuged at 3000 x g for 10 min. The supernatants were retained as samples to be tested. The pellets were washed at neutral pH, and then resuspended in 50 mM sodium carbonate buffer, pH 10.0, with 2 mM DTT and incubated at 37°C for 60 min., to solubilize proteins at high pH.

### Example 2. Preparation of Target Samples from Lepidopteran (Ostrinia nubilalis, Heliothis virescens, Heliothis zea) and Coleopteran (Diabrotica virgifera) Pests

Lepidopteran and coleopteran brush border membrane vesicles (BBMVs) were prepared using a modified version of the method of MacIntosh *et al.* (1994) *J. Inv. Path.* 63:97-98. Second to third instar larvae were homogenized using a motorized tissue grinder in 1x MEH Buffer (300 mM mannitol, 5 mM EGTA, 17 mM HEPES pH 7.5). The crude homogenate was centrifuged at 1000 x g for 15 min at 4°C. The supernatant was passed through Miracloth^{™} (Cal Biochem) into a glass homogenizer with a Teflon pestle and homogenized by hand for 75 strokes. An equal volume of 24 mM MgCl₂ was added to the sample and it was incubated at 4°C, followed by centrifugation at 2500 x g for 15 minutes. The pellet was discarded. The supernatant was centrifuged at 30,000 x g for 30 min. The pellet was resuspended in MEH buffer, and homogenized using a motorized tissue grinder, followed by 10 strokes in a glass homogenizer. An equal volume of 24 mM MgCl₂ was added, and the sample was incubated on ice for 15 min. The sample was centrifuged at 2500 x g for 15 min, and the resulting supernatant was centrifuged at 30,000 x g for 30 min. The pellet was resuspended in 0.5X MEH, homogenized for a few strokes in a glass homogenizer, aliquotted, and stored at -80°C. The BBMVs were quantitated both by protein concentration (Bradford assay) and by endogenous alkaline phosphatase activity (lepidopteran pests) or acid phosphatase activity (*D. virgifer*). Alkaline phosphatase activity was measured by adding known volumes of BBMV suspensions to 200 µl of 1 mg/ml pNPP substrate in 50 mM sodium carbonate, 1 mM MgCl₂, pH 9.8 in microtiter plate wells, incubating the plate for a known amount of time, and reading the absorbance at 405 nm. Acid phosphatase activity was measured by adding known volumes of BBMV suspensions to 50 µl of 7.6 mM pNPP substrate in 90 mM citric acid pH 4.8 in microtiter plate wells, incubating the plate for a known amount of time, stopping the reaction by adding 200 µl 0.1 M NaOH, and reading the absorbance at 405 nm.

### Example 3. Preparation of Target Samples From Soybean Cyst Nematode (Heterodera glycines)

*H. glycines* (race OP50) were grown on soybean as known in the art, and cysts were harvested. Cysts were ruptured with a glass homogenizer, and eggs were hatched on Kimwipes suspended on plastic mesh in a bowl of water. J2 nematodes were collected from the water by centrifugation. Membrane vesicles from the intestinal lining were prepared by sonicating the nematodes 5 -6 times for 10 seconds with 2 minutes of cooling between bursts. The vesicles were then prepared as described for BBMVs in Example 2, with the exception that the sonication substituted for the use of a motorized homogenizer, and the sample was not filtered through Miracloth. The vesicles were quantitated both by protein concentration (Bradford assay) and by endogenous acid phosphatase activity. Acid phosphatase activity was measured as described in Example 2.

### Example 4. Preparation of Target Samples from Caenorhabditis elegans

*C. elegans* strain N2 was grown in liquid and/or on agar plates, and fed with *E*. *coli* (HB101), as known in the art. Nematodes were harvested, and washed to remove the *E coli.* The preparation of BBMVs was as described in example 3. The vesicles were quantitated both by protein concentration (Bradford assay) and by endogenous acid phosphatase activity. Acid phosphatase activity was measured as in Example 2.

### Example 5. Labeling BBMVs by Crosslinking Horseradish Peroxidase, Biotin, and a Fluorescent Dye to BBMV Proteins

BBMVs were labeled with a succinimidyl ester of a fluorescent dye (Alexa-Fluor 488, Molecular Probes, Inc.) following the manufacturer's instructions. After labeling, excess labeling reagent was removed by centrifuging the BBMVs at 30,000 x g for 30 minutes and resuspending them in fresh buffer.

BBMVs were also labeled with maleimide activated horseradish peroxidase (Pierce Biotechnology Inc.) according to the manufacturer's instructions, or with a sulfo-NHS ester of biotin (Pierce Biotechnology Inc.) according to the manufacturer's instructions for detection with avidin conjugated to horseradish peroxidase or to a fluorescent dye.

### Example 6. Labeling of BBMVs or hitestinal Lining Vesicles with Lipophilic Dyes

3,3'-dioctadecyloxacarbocyanine perchlorate (DiO, also known as DiOC₁₈(3)) or 1,1'-dioctadecyl-3,3,3',3'- tetramethylindodicarbocyanine perchlorate (DiD, also known as DiIC₁₈(5)), both obtained in solution from Molecular Probes, Inc. were used to label BBMVs or intestinal lining vesicles as follows. 5 µl of the lipophilic dye solution was added to 1 ml of BBMVs (at a protein concentration of 0.5 mg/ml -1.0 mg/ml), and incubated at 4°C overnight with gentle mixing. The unincorporated label was removed by pelleting the BBMVs at 30,000 x g for 30 min at 4°C, resuspending the BBMVs, and pelleting them again.

### Example 7. Filter-based (Nitrocellulose) Assay for Detection of Strains Having Activity Against Ostrinia nubilalis and Heliothis virescens

### Method 1:

A 5 µl slot pin tool was used to spot bacterial protein samples (see Example 1) that had been treated with trypsin to activate *Cry* toxins onto a dry nitrocellulose filter. Before the spots dried, the filter was placed into blocking buffer (150 mM mannitol, 50 mM CAPS, 2.5 mM EGTA, 4% BSA, pH 10.5) and incubated overnight at 4°C with gentle shaking. The filter was incubated in 2.5 ml BBMV suspension (400 AU/min/ml of alkaline phosphatase activity in blocking buffer) in a sealed plastic bag for 1.5 hrs at room temperature. The filter was then washed three times in 50 ml of blocking buffer without BSA for 5 min at room temperature. The membrane was developed in 15 ml of Bio-Rad BCIP/NBT alkaline phosphatase detection solution for 1-2 hrs.

Table 1 shows the lowest concentration of trypsinized bacterial samples needed to detect the interaction with BBMVs (range tested: 3 µg to 0.3 ng). Bacterial samples positive for binding to BBMVs from *Ostrinia nubilalis* and *Heliothis virescens* also showed insecticidal activity against those organisms when tested in a bioassay.

**Table 1. Detection of BBMV Binding Activity from Bacterial Samples**

| Strain Number | *Ostrinia nubilalis* | *Heliothis virescens* | Insecticidal Activity on *H. virescens* and *O. nubilalis* |
|---|---|---|---|
| ATX13009 | 0.3 µg | 0.03 µg | yes |
| ATX13010 | 0.3 µg | 0.3 µg | yes |
| ATX13011 | 0.03 µg | 0.03 µg | yes |
| ATX13000 | not detected | not detected | no (negative control) |

### Method 2:

Bacterial protein samples (see Example 1) were applied to nitrocellulose filters over a range of 200 ng to 5 µg per spot using a vacuum manifold. The filters were incubated overnight at 4°C in blocking buffer (150 mM mannitol, 50 mM CAPS, 2.5 mM EGTA, 4% BSA, pH 10.5). The filters were then incubated with DiD-labeled BBMVs in the dark in blocking buffer overnight at 4°C with gentle shaking. The filters were washed twice with wash buffer (150 mM mannitol, 50 mM CAPS, 2.5 mM EGTA, pH 10.5) for 5 minutes in the dark. The filters were scanned with a fluorescence imager (Amersham Pharmacia Storm System) to visualize the DiD-labeled BBMVs, and then developed for endogenous alkaline phosphatase in 15 ml of Bio-Rad BCIP/NBT alkaline phosphatase detection solution for 1-2 hrs.

Protein samples from strain ATX13009 bound BBMVs from *H. virescens.* Strain ATX13000 (a negative control strain) did not show binding in this assay. In addition, protein samples from strain ATX13009 were active in *H. virescens* bioassay, while the samples from strain ATX13000 were not active. *H. virescens* and *Ostrinia nubilalis*-binding strains that were identified by the filter method were tested in a bioassay, and found to have pesticidal activity (see Table 2).

**Table 2. Detection of Insecticidal Activity in Strains Identified by the Filter Method**

| Strain Number | Insecticidal Activity on *H*. *virescens* and *O. nubilalis* |
|---|---|
| ATX13009 | yes |
| ATX13010 | yes |
| ATX13011 | yes |
| ATX13000 | no (negative control) |

### Example 8. Plate-based Assay for Detection of Strains Capable of Binding Ostrinia nubilalis or Heliothis virescens BBMVs

Dilutions of bacterial samples (see Example 1) were prepared from 3 µg/100 µl to 0.3 µg/100 µl. 100 µl of each dilution was placed into the wells of a 96-well plate. The plate was incubated 1 hr at room temperature with gentle shaking to bind the bacterial proteins to the plate (if appropriate, the plate was exposed to UV light in a Stratalinker to crosslink proteins to the plate). The liquid was decanted, 200 µl of blocking buffer was added to each well (150 mM mannitol, 50 mM CAPS, 2.5 mM EGTA, 4% BSA, pH 10.5), and the plate was incubated 30 min at room temperature with gentle shaking.

The blocking solution was decanted, and 100 µl of BBMV suspension in blocking buffer was added to each well (400 AU/min/ml alkaline phosphatase activity for *Ostrinia nubilalis* BBMVs or 1700AU/min/ml alkaline phosphatase activity for *Heliothis virescens* BBMVs). The plate was incubated 1.5 hrs at room temperature with gentle shaking. The BBMV suspension was decanted, and the wells were washed 3 times with 200 µl of blocking buffer without BSA. BBMV binding to immobilized proteins was determined by assaying for endogenous alkaline phosphatase activity by adding 200 µl of 1 mg/ml pNPP substrate in 50 mM sodium carbonate, 1 mM MgCl₂, pH 9.8 to each well, incubating the plate up to 16 hrs at room temperature and reading the absorbance at 405 nm.

Binding of *H. virescens* BBMVs to bacterial protein samples was detected using both Universal Covalent (UV crosslinked) and HighBind plates. A signal was detected with 3, 0.3 and 0.03 µg of strain ATX13009 solubilized at high pH on both types of plates. No detectable signal was present from a negative control strain (ATX13000) solubilized at high pH. Strains identified by the plate method were tested in a bioassay against *H. virescens,* and found to be pesticidal (see Table 3).

**Table 3. Insecticidal Activity in Strains identified by the Plate Method**

| Strain Number | Insecticidal Activity on *H. virescens* |
|---|---|
| ATX13009 | yes |
| ATX13000 | no (negative control) |

### Example 9. Binding of AXMI-004 Insecticidal Protein to BBMVs from Ostrinia Nubilis

AXMI-004 is an insecticidal protein active on *Ostrinia nubilalis.* Protein extracts containing AXMI-004 were prepared from pAX920 (a plasmid allowing *Bacillus* expression of proteins) as described in U.S. Patent Application Serial No. 10/782,020. Various amounts of AXMI-004 protein were placed on a filter, and tested for the ability to bind BBMVs of *Ostrinia nubilalis* essentially as described in Example 7. Binding of BBMVs was detected for all of the concentrations tested (see Figure 1A). Negative control strains showed no binding to BBMVs.

### Example 10. Identification of Strains Having Activity Against Western Corn Rootworm (Diabrotica virgifera)

Test samples of bacterial strain 9915 were bound to nitrocellulose filters, which were incubated with *D. virgifera* BBMVs as described above in Example 7. The filters were scanned with a fluorescence imager (Amersham Pharmacia Storm System) to visualize the DiD-labeled BBMVs, then developed for endogenous acid phosphatase in 15 ml of 50 mM trisodium citrate pH 5.6, 10 mg/ml naphthyl phosphate, 0.7 mg/ml fast red TR for 3-4 hours.

The binding activity of strain 9915 on *D. virgifera* BBMVs was greater than controls. Protein samples from strain 9915 exhibited intermittent activity in *D*. *virgifera* bioassays.

### Example 11. Detection of Interactions Between Test Samples and H. glycines Intestinal Lining Vesicles, and Identification of a Strain Having Activity Against H. glycines

Test samples from various bacterial strains were prepared from culture supernatants and high pH-solubilized proteins as described in Example 1. The samples (200 ng to 2 µg of protein) were applied to nitrocellulose filters using a vacuum manifold as described in Example 7. Filters were then blocked in blocking buffer (150 mM Mannitol, 2.5 mM EGTA, 50 mM PIPES, 4% BSA, pH 6.5) overnight at 4°C. The target samples consisted of vesicles prepared from *H. glycines* intestinal lining as described in Example 3, labeled with DiD as described in Example 6. The nitrocellulose filters were incubated with the labeled vesicles in blocking buffer overnight at 4°C in the dark. The filters were washed in wash buffer (150 mM mannitol, 2.5 mM EGTA, 50 mM PIPES, pH 6.5). The filters were then scanned with a fluorescence imager (Amersham Pharmacia Storm System) to visualize the DiD-labeled vesicles, and then developed for endogenous acid phosphatase using 10 mg/ml naphthyl phosphate, 0.7 mg/ml Fast Red TR in 50 mM trisodium citrate pH 5.6 for 4 hours. Strains that reproducibly bound *H. glycines* vesicles were identified. Furthermore, protein samples from these strains showed similar protein profiles on SDS PAGE gels, suggesting that similar binding proteins (potential toxins) were contained in these strains.

Strains used in the vesicle binding assay were tested in an *H. glycines* bioassay. The culture supernatant of a strain that was repeatedly positive in the vesicle binding assay showed bioactivity against *H. glycines,* while strains that were repeatedly negative in the vesicle binding assay did not show bioactivity, as shown in Table 4.

**Table 4. Correlation between Vesicle Binding and Bioassay Activity**

| **Strain** | **Initial vesicle binding assay result** | **Vesicle binding retest result** | ***H. glycines* bioassay result** |
|---|---|---|---|
| | | | **(% mortality)** |
| ATX18930 | positive | positive | >75% |
| ATX18936 | negative | negative | <10% |
| ATX19442 | negative | negative | <10% |
| ATX19509 | negative | negative | <10% |

### Example 12. Detection of Interactions Between Test Samples and C. elegans BBMVs

Bacterial protein samples were prepared from strains ATX9387, ATX7052, ATX 9915, ATX16065, ATX15242, and applied to nitrocellulose filters as described above over a range of 10 ng to 5 µg per spot using a vacuum manifold. The filters were incubated overnight at 4°C in blocking buffer (150 mM mannitol, 50 mM HEPES, 2.5 mM EGTA, 4% BSA, pH 7.5). The nitrocellulose filters were incubated with DiD-labeled vesicles in blocking buffer overnight at 4°C in the dark with gentle shaking. The filters were washed twice with wash buffer (150 mM mannitol, 50 mM HEPES, 2.5 mM EGTA, pH 7.5) for 5 minutes. The filters were then scanned with a fluorescence imager (Amersham Pharmacia Storm System) to visualize the DiD-labeled vesicles, and then developed for endogenous acid phosphatase using 10 mg/ml naphthyl phosphate, 0.7 mg/ml Fast Red TR in 50 mM trisodium citrate pH 5.6 for 4 hours. Protein samples from strains ATX 9387 and ATX 7052 were found to bind *C*. *elegans* BBMVs, whereas protein samples from ATX 9915, ATX 16065, and ATX15242 were not found to bind to *C. elegans* BBMVs (see Figure 1B). Protein samples from ATX9387 and ATX7052 were active in *C. elegans* bioassay.

### Example 13. Bioassays for Pesticidal Activity

The ability of a pesticidal protein to act as a pesticide upon a pest is often assessed in a number of ways. One way well known in the art is to perform a feeding assay. In such a feeding assay, one exposes the pest to a sample containing either compounds to be tested, or control samples. Often this is performed by placing the material to be tested, or a suitable dilution of such material, onto a material that the pest will ingest, such as an artificial diet. The material to be tested may be composed of a liquid, solid, or slurry. The material to be tested may be placed upon the surface and then allowed to dry. Alternatively, the material to be tested may be mixed with a molten artificial diet, then dispensed into the assay chamber. The assay chamber may be, for example, a cup, a dish, or a well of a microtiter plate.

Assays for sucking pests (for example aphids) may involve separating the test material from the insect by a partition, ideally a portion that can be pierced by the sucking mouth parts of the sucking insect, to allow ingestion of the test material. Often the test material is mixed with a feeding stimulant, such as sucrose, to promote ingestion of the test compound.

Other types of assays can include microinjection of the test material into the mouth, or gut of the pest, as well as development of transgenic plants, followed by test of the ability of the pest to feed upon the transgenic plant. Plant testing may involve isolation of the plant parts normally consumed, for example, small cages attached to a leaf, or isolation of entire plants in cages containing insects.

Other methods and approaches to assay pests are known in the art, and can be found, for example in Robertson, J. L. & H. K. Preisler. 1992. *Pesticide bioassays with arthropods.* CRC, Boca Raton, FL. Alternatively, assays are commonly described in the journals "Arthropod Management Tests" and "Journal of Economic Entomology" or by discussion with members of the Entomological Society of America (ESA).

## Claims

1. A method of screening the proteome of an organism for pesticidal activity, comprising:
a) binding test polypeptides derived from said proteome to a substrate;
b) contacting said test polypeptides with target substances expressed by a pest of interest; and,
c) detecting whether said target substances expressed by said pest of interest are bound to said polypeptides;
wherein said polypeptides derived from said proteome are bound to the substrate without separation of individual polypeptides, wherein binding of said polypeptides to the target substances is associated with pesticidal activity.

2. The method of claim 1, wherein said test polypeptides comprise microbial polypeptides.

3. The method of claim 2, wherein said test polypeptides comprise fungal polypeptides.

4. The method of claim 2, wherein said test polypeptides comprise protozoan polypeptides.

5. The method of claim 2, wherein said test polypeptides comprise bacterial polypeptides.

6. The method of claim 2, wherein said microbial polypeptides are isolated from a microbe selected from the group consisting of *Bacillus, Streptomyces, Serratia, Enterobacter, Yersinia, Xenorhabdus, Pseudomonas, Clavibacter, Photorhabdus*, *Paenibacillus,* and *Rhizobium.*

7. The method of claim 6, wherein said microbial polypeptides are from *Bacillus thuringiensis.*

8. The method of claim 1, wherein said test polypeptides comprise plant polypeptides.

9. The method of claim 1, wherein said target substances expressed by said pest of interest are present in brush border membrane vesicles (BBMVs) or intestinal lining membrane vesicles.

10. The method of claim 9, wherein a marker is incorporated into said BBMVs or intestinal lining membrane vesicles.

11. The method of claim 10, wherein said marker is selected from the group consisting of biotin, a lipophilic dye, a fluorescent dye, a colored dye, an antigen, a cofactor, and an enzyme.

12. The method of claim 1, wherein said substrate is a filter.

13. The method of claim 1, wherein said substrate is a microtiter plate.

14. The method of claim 1, wherein said pest of interest is selected from the group consisting of insects, fungi, nematodes, mites, and ticks.

15. The method of claim 14, wherein said pest is a nematode selected from the group consisting of a cyst nematode, a root knot nematode, and a lesion nematode.

16. The method of claim 14, wherein said pest is an insect selected from the group consisting of a corn rootworm, *Heliothis virescens,* a European corn borer, a corn earworm, a lygus bug, an aphid, a black cutworm, a boll weevil, a boll worm, a bud worm, a pink bollworm, a beet armyworm, a fall armyworm, and *Spodoptera frugiperda.*

17. The method of claim 14, wherein said pest is a fungus selected from the group consisting of *Sclerotium, Phytophthora, Fusarium, Pythium, Pseudomonas,* and *Xanthomonas.*

18. The method of claim 1, further comprising performing a bioassay to confirm pesticidal activity of said organism.

19. The method of claim 1, wherein said test polypeptides are recombinant polypeptides.

## Patentansprüche

1. Verfahren zur Durchmusterung des Proteoms eines Organismus auf pestizide Aktivität, umfassend:
a) Binden von Testpolypeptiden, die aus dem Proteom stammen, an ein Substrat;
b) Inkontaktbringen der Testpolypeptide mit Zielsubstanzen, die von einem Schädling von Interesse exprimiert werden; und
c) Detektieren, ob die Zielsubstanzen, die von dem Schädling von Interesse exprimiert werden, an die Polypeptide gebunden werden;
wobei die aus dem Proteom stammenden Polypeptide ohne Abtrennung individueller Polypeptide an das Substrat gebunden werden, wobei die Bindung der Polypeptide an die Zielsubstanzen mit pestizider Aktivität assoziiert ist.

2. Verfahren nach Anspruch 1, wobei die Testpolypeptide mikrobielle Polypeptide umfassen.

3. Verfahren nach Anspruch 2, wobei die Testpolypeptide pilzliche Polypeptide umfassen.

4. Verfahren nach Anspruch 2, wobei die Testpolypeptide Protozoen-Polypeptide umfassen.

5. Verfahren nach Anspruch 2, wobei die Testpolypeptide bakterielle Polypeptide umfassen.

6. Verfahren nach Anspruch 2, wobei die mikrobiellen Polypeptide aus einer Mikrobe, ausgewählt aus der Gruppe, bestehend aus *Bacillus, Streptomyces, Serratia, Enterobacter, Yersinia, Xenorhabdus, Pseudomonas, Clavibacter, Photorhabdus, Paenibacillus* und *Rhizobium,* isoliert sind.

7. Verfahren nach Anspruch 6, wobei die mikrobiellen Polypeptide von Bacillus thuringiensis sind.

8. Verfahren nach Anspruch 1, wobei die Testpolypeptide Pflanzenpolypeptide umfassen.

9. Verfahren nach Anspruch 1, wobei die von dem Schädling von Interesse exprimierten Zielsubstanzen in Bürstensaum-Membranvesikeln (BBMVs) oder Intestinalauskleidungsmembranvesikeln ("intestinal lining membrane vesicles") vorhanden sind.

10. Verfahren nach Anspruch 9, wobei der Marker in die BBMVs oder die Intestinalauskleidungsmembranvesikel eingebaut ist.

11. Verfahren nach Anspruch 10, wobei der Marker ausgewählt ist aus der Gruppe, bestehend aus Biotin, einem lipophilen Farbstoff, einem Fluoreszenzfarbstoff, einem farbigen Farbstoff, einem Antigen, einem Co-Faktor und einem Enzym.

12. Verfahren nach Anspruch 1, wobei das Substrat ein Filter ist.

13. Verfahren nach Anspruch 1, wobei das Substrat eine Mikrotiterplatte ist.

14. Verfahren nach Anspruch 1, wobei der Schädling von Interesse ausgewählt ist aus der Gruppe, bestehend aus Insekten, Pilzen, Nematoden, Milben und Zecken.

15. Verfahren nach Anspruch 14, wobei der Schädling eine Nematode ist, ausgewählt aus der Gruppe, bestehend aus einer Zystennematode, einer Wurzelgallennematode und einer wandernden Wurzelnematode ("lesion nematode").

16. Verfahren nach Anspruch 14, wobei der Schädling ein Insekt ist, ausgewählt aus der Gruppe, bestehend aus einer Blattkäferlarve der Gattung *Diabrotica* ("corn rootworm"), *Heliothis virescens,* Maiszünsler, Baumwollkapselwurm ("corn earworm"), einem Käfer der Gattung Lygus, einer Blattlaus, einer Ypsilon-Eule, einem Baumwollkapselkäfer, einem Kapselwurm, einem Knospenwurm, einem roten Baumwollkapselwurm, einem Heerwurm bzw. *Spodoptera exigua* ("beet armyworm"), einem Herbst-Heerwurm bzw. *Spodoptera frugiperda* ("fall armyworm") und *Spodoptera frugipderda.*

17. Verfahren nach Anspruch 14, wobei der Schädling ein Pilz ist, ausgewählt aus der Gruppe, bestehend aus *Sclerotium, Phytophthora, Fusarium, Pythium, Pseudomonas* und *Xanthomonas.*

18. Verfahren nach Anspruch 1, weiterhin umfassend die Durchführung eines Bioassays zum Bestätigen der pestiziden Aktivität des Organismus.

19. Verfahren nach Anspruch 1, wobei die Testpolypeptide rekombinante Polypeptide sind.

## Revendications

1. Procédé de criblage du protéome d'un organisme pour une activité pesticide, consistant à:
a) lier des polypeptides de test dérivés dudit protéome à un substrat;
b) mettre en contact lesdits polypeptides de test avec des substances cibles exprimées par un ravageur d'intérêt; et
c) détecter si lesdites substances cibles exprimées par ledit ravageur d'intérêt sont liées auxdits polypeptides; dans lequel lesdits polypeptides dérivés dudit protéome sont liés au substrat sans séparation des polypeptides individuels, dans lequel la liaison desdits polypeptides aux substances cibles est associée à l'activité pesticide.

2. Procédé de la revendication 1, dans lequel lesdits polypeptides de test comprennent des polypeptides microbiens.

3. Procédé de la revendication 2, dans lequel lesdits polypeptides de test comprennent des polypeptides fongiques.

4. Procédé de la revendication 2, dans lequel lesdits polypeptides de test comprennent de polypeptides de protozoaires.

5. Procédé de la revendication 2, dans lequel lesdits polypeptides de test comprennent des polypeptides bactériens.

6. Procédé de la revendication 2, dans lequel lesdits polypeptides microbiens sont isolés à partir d'un microbe sélectionné dans le groupe constitué par *Bacillus, Streptomyces, Serratia, Enterobacter, Yersinia, Xenorhabdus, Pseudomonas, Clavibacter, Photorhabdus, Paenibacillus,* et *Rhizobium.*

7. Procédé de la revendication 6, dans lequel lesdits polypeptides microbiens proviennent de *Bacillus thuringiensis.*

8. Procédé de la revendication 1, dans lequel lesdits polypeptides de test comprennent des polypeptides de végétaux.

9. Procédé de la revendication 1, dans lequel lesdites substances cibles exprimées par ledit ravageur d'intérêt sont présentes dans les vésicules membranaires de la bordure en brosse (VMBB) ou dans les vésicules de la membrane de revêtement intestinale.

10. Procédé de la revendication 9, dans lequel un marqueur est incorporé dans lesdites VMBB ou dans les vésicules de la membrane de revêtement intestinale.

11. Procédé de la revendication 10, dans lequel ledit marqueur est sélectionné dans le groupe constitué par la biotine, un colorant lipophile, un colorant fluorescent, une teinture colorée, un antigène, un cofacteur et un enzyme.

12. Procédé de la revendication 1, dans lequel ledit substrat est un filtre.

13. Procédé de la revendication 1, dans lequel ledit substrat est une plaque de microtitration.

14. Procédé de la revendication 1, dans lequel ledit ravageur d'intérêt est sélectionné dans le groupe constitué par les insectes, les champignons, les nématodes, les acariens et les tiques.

15. Procédé de la revendication 14, dans lequel ledit ravageur est un nématode sélectionné dans le groupe constitué par un nématode des racines, une nodosité des racines et une anguillule des prairies.

16. Procédé de la revendication 14, dans lequel ledit ravageur est un insecte sélectionné dans le groupe constitué par une chrysomèle de la racine du maïs, *Heliothis virescens,* une pyrale du maïs, une noctuelle de la tomate, une punaise parasite du Picea glauca, un puceron, un ver gris-noir, un charançon de la capsule, un ver de la capsule, une phalène verdoyante, un ver rose du cotonnier, un légionnaire de la betterave, un légionnaire d'automne, et *Spodoptera frugiperda.*

17. Procédé de la revendication 14, dans lequel ledit ravageur est un champignon sélectionné dans le groupe constitué par *Sclerotium, Phytophthora, Fusarium, Pythium, Pseudomonas,* et *Xanthomonas.*

18. Procédé de la revendication 1, consistant en outre à réaliser un bioessai pour confirmer l'activité de pesticide dudit organisme.

19. Procédé de la revendication 1, dans lequel lesdits polypeptides de test sont des polypeptides recombinants.
